# EUROPEAN PATENT APPLICATION

(11) **EP 4 180 121 A1**
(43) Date of publication of application: **17.05.2023**
(21) Application number: 21383033.4
(22) Date of filing: 15.11.2021
(51) Int. Cl.: B01J 31/16, B01J 31/22, A61K 38/00

(54) **COMPOSITION FOR ASSESSING THE PRESENCE OF MICROORGANISMS IN A TEST SAMPLE**

(71) Applicant: Universidad Autónoma de Madrid, 28049 Madrid (ES); Consejo Superior De Investigaciones Científicas, 28006 Madrid (ES)
(72) Inventor: AMO OCHOA, Pilar, 28049 Madrid (ES); MALDONADO, Noelia, 28049 Madrid (ES); BASTIDA CODINA, María Agatha, 28006 Madrid (ES)
(74) Representative: Hoffmann Eitle

(57) **Abstract**

The present invention refers to a specific composition, which functions as an enzyme mimic or artificial enzyme, for assessing the presence of microorganisms in a test sample. On the other hand, the composition can be used as a medicament, preferably in the treatment of infections caused by said microorganisms.

The composition comprises:

a) a coordination polymer which in turn comprises a polymeric crystalline structure with a repeating core characterized by Formula I: M2L2(H20)4 wherein:

i. M is copper (II) metal ion,

ii. L is an aromatic pseudo-peptide characterized by Formula II: H2I-bis-amino acid or Formula III: H2T-bis-amino acid, wherein T is terephthaloyl, I is isophthaloyl, and the amino acid is a L or D amino acid; and

b) H2O2.

## Description

### FIELD OF THE INVENTION

The present invention refers to the medical field. Particularly, the present invention refers to a specific composition, which functions as an enzyme mimic or artificial enzyme, for assessing the presence of microorganisms in a test sample by means of the identification of the inhibition of the microorganism. On the other hand, the composition can be used as a medicament, preferably in the treatment of infections caused by said microorganisms.

### STATE OF THE ART

Pathogenic intracellular bacteria have been proved to trigger numerous chronic or recurrent infectious diseases that pose substantial global public health threats. The main medical route for the treatment of bacteria is antibiotics, but these microorganisms use many mechanisms to survive by becoming resistant to them. For this reason, the discovery and development of alternative antimicrobial strategies are critical.

On the other hand, enzyme mimics (or artificial enzymes) is a branch of biomimetic chemistry, which aims at imitating the function of natural enzymes. An enzyme mimic is a small molecule complex that models the molecular structure, spectroscopic properties, or reactivity of an enzyme, sometimes called bioinspired complexes.

Altogether, from the chemical point of view, coordination polymers (CPs) can be highly interesting in manufacturing artificial enzymes with antimicrobial capacity. They can generate highly reactive oxygen species (ROS) in a controlled way. Indeed, one exciting advantage of the CPs is that the proper selection of the building blocks (metal ion and organic ligand/s) should allow obtaining a material with the desired properties.

Other advantages of the CPs as possible artificial enzymes are their facile preparation, nano-processability, low cost, and superior stability.

So, in summary, such as it is indicated above, there is an unmet need of finding alternative and effective antimicrobial strategies, which can be used, for instance, to assess the presence of pathogenic microorganisms in a test sample by means of the identification of the inhibition of the microorganism or even in the treatment of infections caused by these microorganisms.

The present invention is focused on solving this problem, and a specific composition, which functions as an enzyme mimic or artificial enzyme, is herein provided, which is precisely used for assessing the presence of pathogenic microorganisms by means of the identification of the inhibition of the microorganism. On the other hand, this composition can be used for the treatment of infections caused by these pathogenic microorganisms.

### DESCRIPTION OF THE INVENTION

### Brief description of the invention

Such as has been explained above, the present invention refers to a specific composition, which functions as an enzyme mimic or artificial enzyme, for assessing the presence of microorganisms in a test sample by means of the identification of the inhibition of the microorganism. On the other hand, the composition can be used as a medicament, preferably in the treatment of infections caused by said microorganisms.

Particularly, the present invention shows a composition comprising a specific CP and H₂O₂, which acts as an artificial multi-enzyme (for instance, peroxidase, catalase, or superoxide dismutase) and, consequently, can be used for assessing the presence of peroxidase, catalase, or superoxide dismutase positive microorganisms in a test sample (the composition exerts a growth-inhibiting effect on the microorganism) by means of the identification of the inhibition of the microorganism, or even in the treatment of infections caused by these pathogenic microorganisms.

More specifically, the present invention shows a Cu(II)-amino acid nano-coordination polymer (NCP), which acts as an artificial multi-enzyme (peroxidase, catalase, or superoxide dismutase). Particularly, the 2D CP, with the formula [Cu₂(IBA)₂(H₂O)₄]ₙ·6*ₙ*H₂O is synthesized in one step at sustainable conditions (water and room temperature) by the combination of Cu(II) as metal center with a pseudo amino acid (H₂IBA =Isophthaloyl bis β-alanine). Quantitative total x-ray fluorescence (TXRF) analyses show that the obtained CP is able to release Cu(II) ions slowly. Additionally, this CP can be nano-processed and gelled as a metal-organic gel (MOG) by using different Cu(II) salts concentrations and the application of ultrasounds. Considering its nanometric dimensions, the slow Cu(II) release, and its simple processability, its antibacterial ability was explored to determine its performance as an artificial enzyme. The results show that the compound exhibits antibacterial activity in the presence of hydrogen peroxide, acting as an artificial catalase, peroxidase and SOD with selective behavior for three bacterium strains (*Sphingobacterium spiritivorum, Alcaligenes faecalis* or *Bacillus cereus*). Indeed, it shows a stronger inhibition capacity for *Sphingobacterium.* Additionally, as there are no clinical tests capable of detecting the presence of *Sphingobacterium* in a comfortable, practical, and low-cost way, the compound can be easily embedded to form moldable gelatin that will allow performing tests for the identification of the above microorganisms. The present invention shows that creating new composite materials by combining Cu(II) CPs and organic matrices (such as polyacrylamide, cellulose, fiber, gelatin, chitosan cotton, among others) can improve antibacterial effectiveness in addition to new mechanical properties allowing their manufacture.

So, in summary, the present invention combines Cu(II) ion with a pseudo amino acid (H₂IBA =Isophthaloyl bis β-alanine). The synthesis in a single step and sustainable conditions (water and 25°C) allows obtaining a 2D CP that can be nano-processed and gelled by helping sonication and different Cu(II) metal salts. The obtained CP with the chemical formula [Cu₂(IBA)₂(H₂O)₄]ₙ·6*ₙ*H₂O shows slow Cu(II) release and behaves as peroxidase, catalase, or superoxide dismutase like activity, being particularly effective for *Sphingobacterium.* In addition, its facile application as gel embedded in gelatin could also display some advantages due to its high ability to absorb water and activate macrophages and homeostasis, aiding in the self-assembly of collagen-related peptides and the coagulation of bleeding wounds.

So, the first embodiment of the present invention refers to a composition (hereinafter "composition of the invention") which comprises:
a) A coordination polymer which in turn comprises a polymeric crystalline structure with a repeating core characterized by *Formula I:* **M**₂**L**₂(H₂O)₄ wherein:
   i. **M** is copper (II) metal ion,
   ii. **L** is an aromatic pseudo-peptide characterized by *Formula II:* H₂**I**-bis-**amino acid** or *Formula III:* H₂**T**-bis-**amino acid,** wherein **T** is Terephthaloyl, **I** is Isophthaloyl, and the **amino acid** is a L or D amino acid; and
b) H₂O₂.

In a preferred embodiment, **L** is an aromatic pseudo-peptide selected from the group comprising: H₂**T**-bis-L-aspartate, H₂**T**-bis-D-aspartate; H₂**T**-bis-L-alanine, H₂**T**-bis-D-alanine; H₂**T**-bis-L-valine, H₂**T**-bis-D-valine, H₂**T**-bis-L-isoleucine, H₂**T**-bis-D-isoleucine, H₂**T**-bis-L-leucine, H₂**T**-bis-D-leucine, H₂**T**-bis-L- phenylalanine, H₂**T**-bis-D-phenylalanine, H₂**T**-bis-β-alanine, H₂**T**-bis- β -alanine; H₂**I**-bis β-alanine H₂**I**-bis-L-aspartate, H₂**I**-bis-D-aspartate; H₂**I**-bis-L-alanine, H₂**I**-bis-D-alanine; H₂**I**-bis-L-valine, H₂**I**-bis-D-valine, H₂**I**-bis-L-isoleucine, H₂**I**-bis-D-isoleucine, H₂**I**-bis-L-leucine, H₂**I**-bis-D-leucine, H₂**I**-bis-L-phenylalanine, H₂**I**-bis-D-phenylalanine, H₂**I**-bis-β-alanine or H₂**T**-bis-β-alanine, wherein **T** is Terephthaloyl, **I** is Isophthaloyl.

In a particularly preferred embodiment, the composition of the invention comprises:
a) A coordination polymer which in turn comprises a polymeric crystalline structure with a repeating core characterized by *Formula I:* **M**₂**L**₂(H₂O)₄ wherein:
   i. **M** is copper (II) metal ion,
   ii. **L** is H₂-Isophthaloyl-bis-β-alanine (IBA); and
b) H₂O₂.

In a preferred embodiment, the polymeric crystalline structure of *Formula I* is in the form of composite material, wherein the polymeric crystalline structure of *Formula I* is embedded in an organic matrix.

In a preferred embodiment, the organic matrix is selected from: polyethylene glycol, agarose, polyacrylamide, cellulose, fiber, gelatin, or chitosan.

In a preferred embodiment, the composition is characterized in that the polymeric crystalline structure of *Formula I* is in the form of a mouldable gelatin.

The second embodiment of the present invention refers to a pharmaceutical composition comprising the composition of the invention and, optionally, pharmaceutically acceptable excipients or carriers.

The third embodiment of the present invention refers to the use of the composition of the invention for assessing the presence of a catalase-positive microorganism in a test sample by means of the identification of the inhibition of the microorganism, wherein the composition exerts a growth-inhibiting effect on the microorganism.

In a preferred embodiment, the present invention refers to the use of the composition of the invention for assessing the presence of a catalase and oxidase-positive aerobic or anaerobic microorganism in a test sample by means of the identification of the inhibition of the microorganism, wherein the composition exerts a growth-inhibiting effect on the microorganism.

In a preferred embodiment, the catalase-positive microorganism is a bacterium pertaining to a genus selected from: *Sphingobacterium, Alcaligenes,* or *Bacillus.*

In a preferred embodiment, the catalase-positive microorganism is a bacterium selected from: *Sphingobacterium spiritivorum, Alcaligenes faecalis,* or *Bacillus cereus.*

The fourth embodiment of the present invention refers to a method for assessing the presence of a catalase-positive microorganism in a test sample by means of the identification of the inhibition of the microorganism, which comprises:
a) Spreading the composition of the invention over the test sample, and
b) Wherein the identification of a zone of inhibition in the test sample is indicative of the inhibition of the microorganism.

In a preferred embodiment, the present invention refers to a method for assessing the presence of a catalase and oxidase aerobic or anaerobic microorganism in a test sample by means of the identification of the inhibition of the microorganism, which comprises:
a) Spreading the composition of the invention over the test sample, and
b) Wherein the identification of a zone of inhibition in the test sample is indicative of the inhibition of the microorganism.

In a preferred embodiment, the present invention refers to a method for assessing the presence of a bacterium pertaining to a genus selected from: *Sphingobacterium, alcaligenes* or *Bacillus,* preferably a bacterium selected from: *Sphingobacterium spiritivorum, Alcaligenes faecalis* or *Bacillus cereus* by means of the identification of the inhibition of the microorganism.

The last embodiment of the present invention refers to a pharmaceutical composition comprising the composition of the invention, and optionally pharmaceutically acceptable excipients or carriers, for use as a medicament, preferably in the treatment of a bacterial infection caused, for example, by a bacterium pertaining to a genus selected from: *Sphingobacterium, alcaligenes* or *Bacillus,* preferably a bacterium selected from: *Sphingobacterium spiritivorum, Alcaligenes faecalis* or *Bacillus cereus.* Alternatively, this embodiment refers to a method for treating a patient suffering from a bacterial infection caused, for example, by a bacterium pertaining to a genus selected from: *Sphingobacterium, Alcaligenes,* or *Bacillus,* preferably a bacterium selected from: *Sphingobacterium spiritivorum, Alcaligenes faecalis,* or *Bacillus cereus,* which comprises the administration of a therapeutically effective dose or amount of the pharmaceutical composition of the invention.

For the purpose of the present invention, the following terms are defined:
- The term "comprising" means including, but not limited to, whatever follows the word "comprising". Thus, the use of the term "comprising" indicates that the listed elements are required or mandatory but that other elements are optional and may or may not be present.
- The term "consisting of" means including, and limited to, whatever follows the phrase "consisting of". Thus, the phrase "consisting of" indicates that the listed elements are required or mandatory and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of the pharmaceutical composition of the invention is intended an amount that, when administered to the subject, brings about a positive therapeutic response in a subject having a bacterial infection. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- Abbreviations: CP, coordination polymer; NCP, nano-coordination polymer; MOG, Metal-organic gel; TMB, 3,3',5,5'-Tetramethylbenzidine; NBT, nitroblue benzolium; TA, Therephtalic acid; PBS, phosphate-buffered saline; H₂IBA, isophthaloyl bis β-alanine; ATCC, American Type Culture Collection; LB broth, Luria Bertani broth; M-H broth, Mueller Hilton broth; PXRD, Powder X-ray diffraction; TGA, Thermogravimetric analysis; TXRF, Total x-ray fluorescence; FESEM, Field emission scanning electron microscopy; AFM, Atomic force microscopy; XPS, X-ray photoelectron spectroscopy, ROS, Reactive oxygen species; SOD, superoxo-dismutase; OD, optical density, AF, antiferromagnetic; MIC, minimal inhibitory concentration.

### Description of the figures

**Figure 1****.** IR spectra of **[Cu₂(IBA)₂(H₂O)₄]ₙ·6*ₙ*H₂O** where black line represents bulk **[Cu₂(IBA)₂(H₂O)₄|n·6*ₙ*H₂O** and green line **NCP-[Cu₂(IBA)₂(H₂O)₄]ₙ·6*ₙ*H₂O.**
**Figure 2****.** PXRD patterns of **[Cu₂(IBA)₂(H₂O)₄]ₙ·6*ₙ*H₂O.** The blue line corresponds to the experimental data, and the black line corresponds to simulated data.
**Figure 3****.** PXRD patterns of **[Cu₂(IBA)₂(H₂O)₄]ₙ·6*ₙ*H₂O.** The blue line corresponds to **bulk [Cu₂(IBA)₂(H₂O)₄]ₙ·6*ₙ*H₂O,** and the black line corresponds to **NCP-[Cu₂(IBA)₂(H₂O)₄]ₙ·6*ₙ*H₂O.**
**Figure 4****.** Thermal variation of χₘ (left) and χₘT (right) for compound **[Cu₂(IBA)₂(H₂O)₄]ₙ·6*ₙ*H₂O.**
**Figure 5****.** Magnetization (M) variation for compound **[Cu₂(IBA)₂(H₂O)₄]ₙ·6*ₙ*H₂O.**
**Figure 6****.** Photograph of **NCP-[Cu₂(IBA)₂(H₂O)₄]_{n·}6*ₙ*H₂O@gelatin.**
**Figure 7****.** PXRD patterns of pristine compound **NCP-[Cu₂(IBA)₂(H₂O)₄]ₙ·6*ₙ*H₂O** (black line), **NCP-[Cu₂(IBA)₂(H₂O)₄]n·6*ₙ*H₂O@gelatin** (light blue line).
**Figure 8****. NCP-[Cu₂(IBA)₂(H₂O)₄]ₙ·6*ₙ*H₂O** in aqueous solution at pH 3 after (a) 10 min (left) and 2 hours (right); (b) SEM image of **NCP-[Cu₂(IBA)₂(H₂O)₄]ₙ·6*ₙ*H₂O** turned into white solid formed before its total dissolution.
**Figure 9****. NCP-[Cu₂(IBA)₂(H₂O)₄]ₙ·6*ₙ*H₂O** in aqueous solution at pH 9 after (a) 10 min (left) and 2 hours (right); (b) SEM image of degraded solid.
**Figure 10****.** Thermal stability of compound **NCP-[Cu₂(IBA)₂(H₂O)₄]ₙ·6*ₙ*H₂O.** Thermogram signals: black and the red line (weight (TG) and derivate weight (DTG) represent the stages of the losses produced. Multicolor-labeled lines represent the ion current associated with each mass lost in each step.
**Figure 11****.** Colour change of compound **NCP-[Cu₂(IBA)₂(H₂O)₄]ₙ·6*ₙ*H₂O,** from blue to green, when it is heated at 100 °C for 20 min (thermochromism). This green material returns to pristine **NCP-[Cu₂(IBA)₂(H₂O)₄]ₙ·6*ₙ*H₂O** when it is immersed in water (solvatochromism).
**Figure 12****.** PXRD patterns of **[Cu₂(IBA)₂(H₂O)₄]ₙ·6*ₙ*H₂O.** The blue line corresponds to **NCP-[Cu₂(IBA)₂(H₂O)₄]ₙ·6*ₙ*H₂O,** green line corresponds to **NCP-[Cu₂(IBA)₂(H₂O)₄]ₙ·6*ₙ*H₂O** heated at 100 °C, and light-blue line to **NCP-[Cu₂(IBA)₂(H₂O)₄]ₙ·6*ₙ*H₂O** immersed in water after being heated.
**Figure 13****.** Thermal stability of compound **NCP-[Cu₂(IBA)₂(H₂O)₄]ₙ·6*ₙ*H₂O** after being previously heated at 100 °C. Black and red lines represent the weight (TG) and derivate weight (DTG), respectively, showing the stages of the losses produced. Multicolor-labeled lines represent the ion current associated with each mass lost in each stage.
**Figure 14****.** Copper(II) concentration of compound **NCP-[Cu₂(IBA)₂(H₂O)₄]ₙ·6*ₙ*H₂O** in water (a) and PBS (b) after carrying out copper release assays.
**Figure 15****.** Oxygen bubbles generation when **NCP--[Cu₂(IBA)₂(H₂O)₄]ₙ·6*ₙ*H₂O** is exposed to H₂O₂ 30 % for a) t=0, b) t=10 s, c) t=20 s and d) t=30 s.
**Figure 16****.** Absorbance measurement against reaction time when the absorption reaches a maximum.
**Figure 17****.** Measurements of NBT absorption spectra at 560 nm upon 20 min of reaction to different concentrations of **NCP--[Cu₂(IBA)₂(H₂O)₄]ₙ·6*ₙ*H₂O.**
**Figure 18****.** Isophthaloyl bis β-alanine (H₂IBA) coordination modes to Cu(II) metal centers in compound **-[Cu₂(IBA)₂(H₂O)₄]ₙ·6*ₙ*H₂O** (a). Structure Fragment of compound **[Cu₂(IBA)₂(H₂O)₄]ₙ·6*ₙ*H₂O** (b). Water solvation molecules have been omitted for clarity. Blue color corresponds to Cu atoms, red to oxygen atoms; purple to nitrogen atoms; grey to carbon atoms, and white to hydrogen atoms.
**Figure 19****.** SEM images of compound **[Cu₂(IBA)₂(H₂O)₄]ₙ·6*ₙ*H₂O** (a) and **NCP-[Cu₂(IBA)₂(H₂O)₄]ₙ·6*ₙ*H₂O** (c) together with statistical histograms of widths (b, d).
**Figure 20****.** AFM images of compound **NCP-[Cu₂(IBA)₂(H₂O)₄]ₙ·6*ₙ*H₂O** (a) and statistical histogram of height (b).
**Figure 21****.** FESEM images of Sphingobacterium with no treatment (a), after being treated with 100 µL **NCP-[Cu₂(IBA)₂(H₂O)₄]ₙ·6*ₙ*H₂O** + 50 µl H₂O₂ (30 %) for 24 h (b).
**Figure 22****.** (a) Absorbance spectra from 200 to 700 nm of TMB (grey line), TMB + **NCP-[Cu₂(IBA)₂(H₂O)₄]ₙ·6*ₙ*H₂O** (where NCP-[M₂L₂(H₂O)₄] 6H₂O =1n) (red line) and TMB + **NCP-[Cu₂(IBA)₂(H₂O)₄]ₙ·6*ₙ*H₂O** + H₂O₂ (blue line). (b) Time-dependent absorbance changes of oxidized TMB (grey line), TMB + **NCP-[Cu₂(IBA)₂(H₂O)₄]ₙ·6*ₙ*H₂O** (red line) and TMB + **NCP-[Cu₂(IBA)₂(H₂O)₄]ₙ·6*ₙ*H₂O** + H₂O₂ (blue line).
**Figure 23****.** Decrease in NBT absorbance versus progressive increase in **NCP-[Cu₂(IBA)₂(H₂O)₄]ₙ·6*ₙ*H₂O** concentration (0-5 mg/ml) (a) and **NCP-[Cu₂(IBA)₂(H₂O)₄|ₙ·6*ₙ*H₂O** concentration that inhibits the NBT reduction rate by 50% (1.1 mg/ml) (b).
**Figure 24****.** Photographs of *Sphingobacterium spiritovirum* colony (50µL of overnight of culture after 10⁻⁸ dilution) (a) colony count method for the blank experiment (40µL of H₂O₂ at 25mM), 490 colonies (b) colony count method after being treated *Sphingobacterium* with **NCP-[Cu₂(IBA)₂(H₂O)₄]ₙ·6*ₙ*H₂O @gelatin** (40µL at 3mg/ml) plus H₂O₂ (40µL at 25mM), 210 colonies.

### Detailed description of the invention

The present invention is illustrated by means of the Examples set below without the intention of limiting its scope of protection.

### Example 1. Material and methods

### Example 1.1. Materials and Instrumentation

### Example 1.1.1. Materials

All reagents and solvents were purchased from standard chemical suppliers: CuSO₄·5H₂O >98% Prolabo; CuSO₄ anhydrous >99% Panreac; Cu(NO₃)₂·3H₂O extra pure and sodium hydroxide (NaOH) Scharlau; Gelatin Bloon 100-120 Angel Jobal (animal origin); 3,3',5,5'-Tetramethylbenzidine (TMB) Sigma Aldrich; MeOH 99.8% and EtOH 99% Scharlau, and used as received. Phosphate-buffered saline (PBS) 10 mM was prepared as described in standard protocols. Isophthaloyl bis β-alanine (H₂IBA) was synthesized as described in the literature.All microorganisms were from American Type Culture Collection (ATCC), and LB broth-Agar medium was from DifcoTM. LB-Agar was prepared in distilled water (1 L) by dissolving agar (15 g) and LB broth (25 g) autoclaving at 15 psi, from 121-124 °C for 15 minutes.

### Example 1.1.2. Instrumentation

**Infrared (FT-IR) spectra** were recorded on a PerkinElmer 100 spectrophotometer using a PIKE Technologies MIRacle Single Reflection Horizontal ATR Accessory from 4000-500 cm⁻¹.

**Elemental analysis** was performed on an elementary microanalyzer LECO CHNS-932. It works with controlled doses of O₂ and a combustion temperature of 1000 °C.

**Powder X-ray diffraction (PXRD)** was collected using a PANalytical X'Pert PRO MPD θ/2θ secondary monochromator and detector with fast X'Celerator, which was used for general assays. Theoretical X-ray powder diffraction patterns were calculated using Mercury Cambridge Structural Database (CSD) version 4.0.0 software from the Crystallographic Cambridge Database. The samples were analysed with scanning θ/2θ.

**Thermogravimetric analysis (TGA)** was performed on a TGA Q500 Thermobalance with an EGA (Evolved Gas Analysis) furnace and a quadrupole mass spectrometer Thermostat Pfeiffer from Tecnovac, to analyse gases, which are given off from the sample. The powder sample was analysed using a Pt sample holder and N₂ flow as purge gas of 90 mL/min with a heating ramp from room temperature to 1000 °C at 10 °C/min.

**Field emission scanning electron microscopy (FESEM)** images were recorded on a FEI VERIOS 460 and JEOL JSM 7600F field emission scanning electron microscopes. SEM-EDX images and EDX spectra were recorded using a Hitachi S-3000N microscope with an ESED coupled to an INCAx-sight EDX analyzer. For this technique, the samples were metallized with a gold layer of 15 nm, under a pressure of 10⁻³ Pa. Bacteria samples for SEM were rinsed with PBS three times and centrifuged at 7000 rpm for 5 min. Then, they were fixed with 2.5 % glutaraldehyde for 3 h at 4 °C. After that, bacteria were dehydrated in ethanol solutions with a graded series (20-100%) for 10 min each time and stored at 4 °C 48 h. Finally, they were deposited by drop-casting on a SiO₂ surface.

**Atomic Force Microscopy (AFM)** images were acquired in dynamic mode using a Nanotec Electronica system operating at standard conditions. For AFM measurements, Olympus cantilevers were used with a nominal force constant of 0.75 N/m and a resonance frequency of about 70 kHz. The images were processed using WSxM. The surfaces used for AFM were SiO₂ 300 nm thickness (IMS Company). SiO₂ surfaces were sonicated in an ultrasound bath at 37 Khz and 380 Watts, for 15 min in acetone, 15 min in 2-propanol, and then dried under an Argon flow. 40 µL of the sample diluted in Milli-Q water were deposited on the SiO₂ substrate by drop-casting, allowing to adsorb for 15 min at room temperature.

**Magnetic measurements** were done in a Quantum Design MPMS-XL-5 SQUID magnetometer in the 2-300 K temperature range with an applied magnetic field of 0.1 T to the crystalline samples.

Qualitative and quantitative **total x-ray fluorescence (TXRF)** analyses were performed with a benchtop S2 PICOFOX TXRF spectrometer from Bruker Nano (Germany). This is equipped with a Molybdenum X-ray source working at 50 kV and 600 µA, a multilayer monochromator with 80% of reflectivity at 17.5 keV (Mo K_{α}), a XFlash SDD detector, with an effective area of 30 mm², and an energy resolution better than 150 eV for 5.9 keV (Mn K_{α}). The acquisition time for qualitative analysis was 300 s and for the quantitative analysis was 600 s. Titanium was chosen as the internal standard for quantification mainly because this element was not present in the samples and to avoid chemical distortion of the samples and volatilization of Cl and S. The Spectra 7 software from Bruker was used for control, acquisition, deconvolution, and integration of all analyzed samples.

### Example 1.2. Synthesis of [Cu₂(IBA)₂(H₂O)₄]ₙ·6ₙH₂O, NCP-[Cu₂(IBA)₂(H₂O)₄]ₙ·6ₙH₂O and NCP-[Cu₂(IBA)₂(H₂O)₄]ₙ·6ₙH₂O@gelatin

**Example 1.2.1. Synthesis of [Cu₂(IBA)₂(H₂O)₄]ₙ·6*ₙ*H₂O:** A mixture of Cu(NO₃)₂ 3H₂O (0.1 g, 0.4 mmol), H₂IBA (0.127 g, 0.4 mmol), and NaOH (0.033 g, 0.8 mmol) was stirred for 1h at room temperature in 16 ml of MilliQ water (pH=3.4). After that, a blue solid was obtained, filtered off, washed with water, ethanol, and diethyl ether, and dried in air (compound **[Cu₂(IBA)₂(H₂O)₄]ₙ·6*ₙ*H₂O).** After one week, light-blue needle crystals can also be obtained from the mother liquors in a Petri dish at room temperature. Its structure was previously published by S. Lymperopoulou et al. Yield 16 % based on Cu. Anal. Calcd. (found) % for C₂₈H₄₈Cu₂N₆O₂₂: C, 36.56 (36.56); H, 5.26 (5.14); N, 6.09 (6.10). The IR has the following characteristic bands (cm⁻¹): 3387 (m), 3281 (s), 1659 (m), 1623 (s), 1563 (s), 1531 (s), 1407 (w), 1078 (w), 715 (s), 684 (s). PXRD of the solid has been performed and corresponds to compound **[Cu₂(IBA)₂(H₂O)₄]ₙ·6*ₙ*H₂O.**

**Example 1.2.2. Synthesis of NCP**-**[Cu₂(IBA)₂(H₂O)₄]ₙ@MOG and NCP-[Cu₂(IBA)₂(H₂O)₄]ₙ·6*ₙ*H₂O:** A solution of CuSO₄ anhydrous (1.2 g, 7.5 mmol) in 5 ml of MilliQ water was rapidly added to another solution formed by H₂IBA (1.54 g, 5 mmol) and NaOH (0.4 g, 10 mmol) in 11 ml of MilliQ water (pH=3) both at room temperature. Blue fibers were instantly observed. When the mixture was stirring for 5 min, in a sonication bath (37 GHz, 100 % powder), an unstable gel **NCP**-**[Cu₂(IBA)₂(H₂O)₄]ₙ@MOG** was formed. This MOG can be transformed into nanocrystals **(NCP**-**[Cu₂(IBA)₂(H₂O)₄]ₙ·6nH₂O)** after still sonication in the same conditions for 20 min more. Yield 20 % based on Cu. Anal. Calcd. (found) % for C₂₈H₄₈Cu₂N₆O₂₂: C, 36.56 (36.92); H, 5.26 (4.99); N, 6.09 (6.15). The IR has the following characteristic bands (cm⁻¹): 3388 (m), 3286 (s), 1660 (m), 1628 (s), 1561 (s), 1531 (s), 1406 (w), 1080 (w), 720 (s), 684 (s). PXRD of the solid has been performed and corresponds to compound **[Cu₂(IBA)₂(H₂O)₄]ₙ·6*ₙ*H₂O**.

**Example 1.2.3. Synthesis of NCP-[Cu₂(IBA)₂(H₂O)₄]ₙ·6*ₙ*H₂O@gelatin:** 0.21 g of gelatin was dissolved in 2 ml f of Milli-Q water at room temperature. Subsequently, 20.8 mg **of NCP-[Cu₂(IBA)₂(H₂O)₄]ₙ·6*ₙ*H₂O** was added to the solution and stirred for 1 h. The mixture was left to rest at 4 °C for 10 min until it got consistency.

### Example 1.3. Characterization of [Cu₂(IBA)₂(H₂O)₄]ₙ·6ₙH₂O NCP-[Cu₂(IBA)₂(H₂O)₄]ₙ·6ₙH₂O and NCP-[Cu₂(IBA)₂(H₂O)₄]ₙ·6ₙH₂O@gelatin

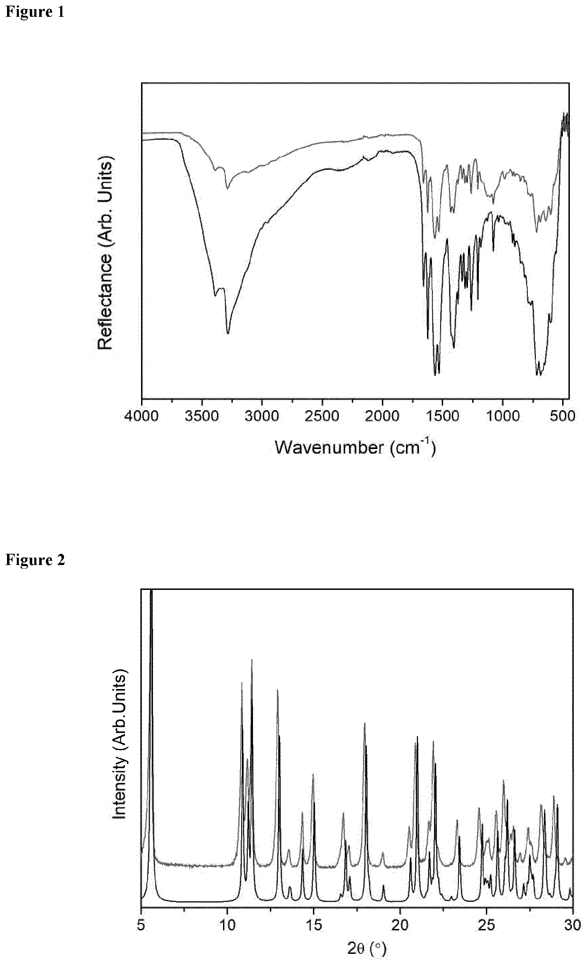

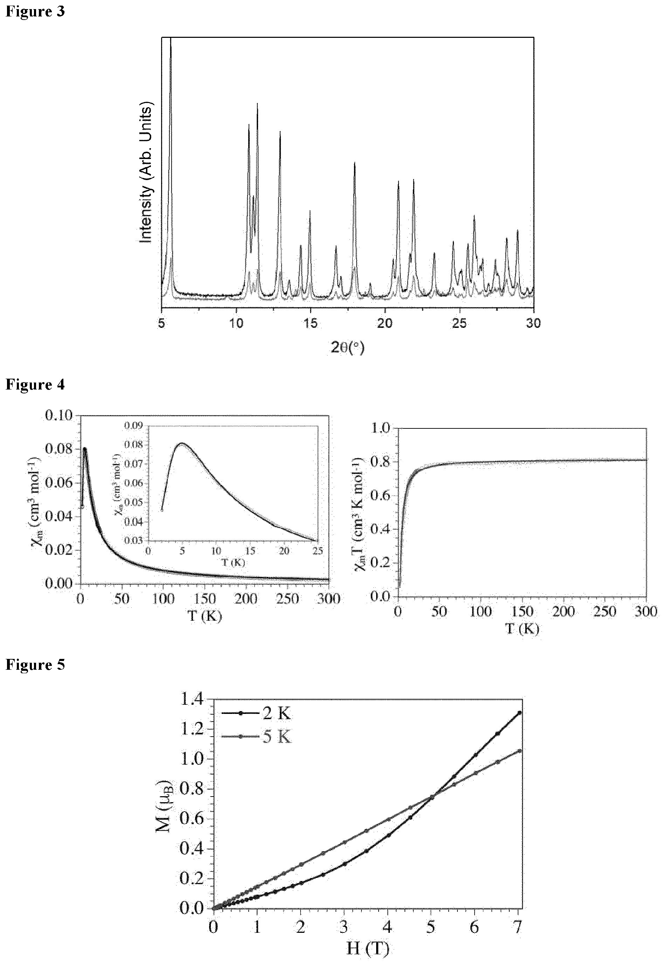

See **Figure 1** showing IR spectra of **[Cu₂(IBA)₂(H₂O)₄]ₙ·6*ₙ*H₂O** where black line represents bulk **[Cu₂(IBA)₂(H₂O)₄]ₙ·6*ₙ*H₂O** and green line **NCP-[Cu₂(IBA)₂(H₂O)₄]ₙ·6*ₙ*H₂O** **Figure 2** showing PXRD patterns of **[Cu₂(IBA)₂(H₂O)₄]ₙ·6*ₙ*H₂O.** The blue line corresponds to the experimental data, and the black line corresponds to simulated data, **Figure 3** showing PXRD patterns of **[Cu₂(IBA)₂(H₂O)₄]ₙ·6*ₙ*H₂O.** The blue line corresponds to **bulk [Cu₂(IBA)₂(H₂O)₄]ₙ·6*ₙ*H₂O,** and the black line corresponds to **NCP-[Cu₂(IBA)₂(H₂O)₄]ₙ·6*ₙ*H₂O** and **Figure 4** showing the thermal variation of χₘ (left) and χₘT (right) for compound **[Cu₂(IBA)₂(H₂O)₄]ₙ·6*ₙ*H₂O.**

A maximum is observed at about 5 K χₘ, which is the typical behavior of an antiferromagnetic (AF) coupling. The χₘT curve shows a progressive decline at low temperatures that is also expected for an AF coupling. It fits to an S = ½ dimer model since the structure shows a Cu(II) dimer connected with a double oxide bridge. The model fits very well with the parameters g = 2.1445, J = -6.0 cm^{-1,} and a monomer impurity of 6.3 %

See **Figure 5** showing magnetization (M) variation for compound **[Cu₂(IBA)₂(H₂O)₄]ₙ·6*ₙ*H₂O.**

At 2 K, magnetization shows a slope change with an inflection point at nearly 5 T. This indicates the compound may be metamagnetic, it means, at low fields the coupling is AF but above the critical field (about 6 T) coupling is ferromagnetic.

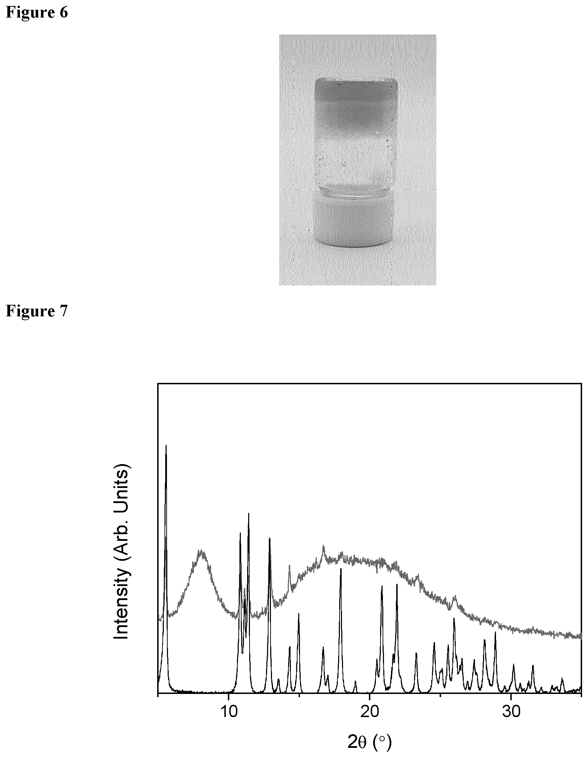

See **Figure 6** showing a photograph of **NCP-[Cu₂(IBA)₂(H₂O)₄]ₙ·6*ₙ*H₂O@gelatin** and **Figure 7** showing PXRD patterns of pristine compound **NCP-[Cu₂(IBA)₂(H₂O)₄]ₙ·6*ₙ*H₂O** (black line), **NCP-[Cu₂(IBA)₂(H₂O)₄]ₙ·6*ₙ*H₂O@gelatin** (light blue line).

### Example 1.4. Solvent and thermal stability of NCP-[Cu₂(IBA)₂(H₂O)₄]ₙ·6ₙH₂O

Compound **NCP-[Cu₂(IBA)₂(H₂O)₄]ₙ·6*ₙ*H₂O** is stable at room temperature in water at neutral pH, however, it turns into a white solid and dissolves at acid pHs (lower than 3), and degrades at basic pHs.

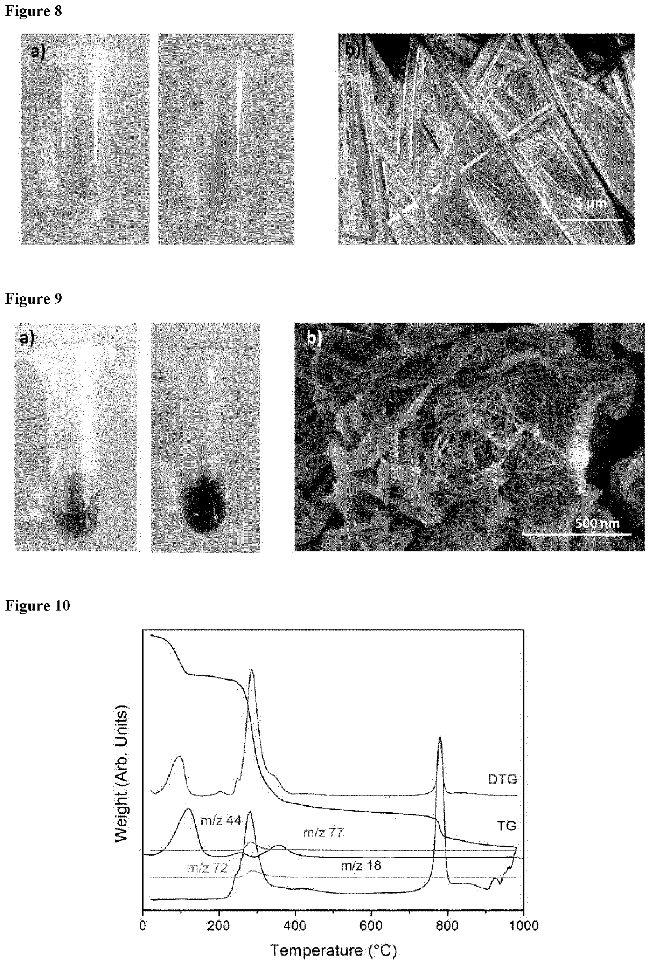

See **Figure 8** showing the stability of **NCP-[Cu₂(IBA)₂(H₂O)₄]ₙ·6*ₙ*H₂O** in aqueous solution at pH 3 after (a) 10 min (left) and 2 hours (right); (b) SEM image of **NCP-[Cu₂(IBA)₂(H₂O)₄]ₙ·6*ₙ*H₂O** turned into white solid formed before its total dissolution and **Figure 9** showing the stability of **NCP-[Cu₂(IBA)₂(H₂O)₄]ₙ·6*ₙ*H₂O** in aqueous solution at pH 9 after (a) 10 min (left) and 2 hours (right); (b) SEM image of degraded solid.

See **Figure 10** showing the thermal stability of compound **NCP-[Cu₂(IBA)₂(H₂O)₄]ₙ·6*ₙ*H₂O** Thermogram signals: black and the red line (weight (TG) and derivate weight (DTG), represent the stages of the losses produced. Multicolor-labeled lines represent the ion current associated with each mass lost in each stage.

All water molecules were lost at 254 °C in three stages. The six solvation molecules further two coordination molecules (obs. 14.97%; calc. 15.2%). The last remaining coordinated two water molecules were lost in two steps with a total weight loss of 3.5 % (cald. 3.8%). After 254 °C, H₂IBA ligands were mainly lost as CO₂ and water (obs. 60.2%; calc. 65%). The loss of fragments such as the aromatic ring (m/z=77) and part of the aliphatic chain (m/z=72) are also appreciated.

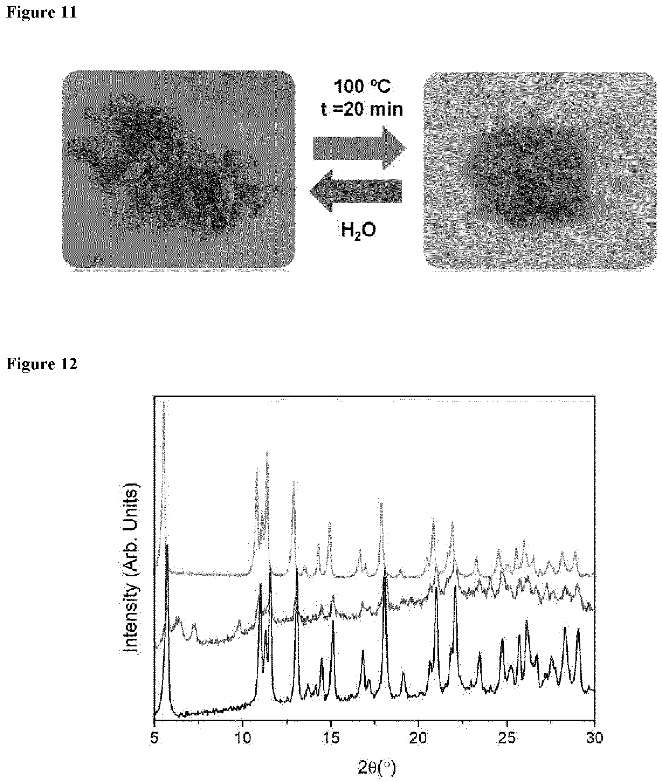

Besides, compound **NCP-[Cu₂(IBA)₂(H₂O)₄]ₙ·6*ₙ*H₂O** presents thermo- and solvatochromic properties leading to a new green less crystalline phase when temperature up 100 °C is applied. Compound **[Cu₂(IBA)₂(H₂O)₄]ₙ·6*ₙ*H₂O** can be recovering from this less crystalline new phase by immersing it in water. See **Figure 11** showing the colour change of compound **NCP-[Cu₂(IBA)₂(H₂O)₄]ₙ·6*ₙ*H₂O,** from blue to green, when it is heated at 100 °C for 20 min (thermochromism). This green material returns to pristine **NCP-[Cu₂(IBA)₂(H₂O)₄]ₙ·6*ₙ*H₂O** when it is immersed in water (solvatochromism), and **Figure 12** showing PXRD patterns of **[Cu₂(IBA)₂(H₂O)₄]ₙ·6*ₙ*H₂O.** The blue line corresponds to **NCP-[Cu₂(IBA)₂(H₂O)₄]ₙ·6*ₙ*H₂O,** green line corresponds to **NCP-[Cu₂(IBA)₂(H₂O)₄]ₙ** heated at 100 °C, and light-blue line to **[Cu₂(IBA)₂(H₂O)₄]ₙ·**immersed in water after being heated.

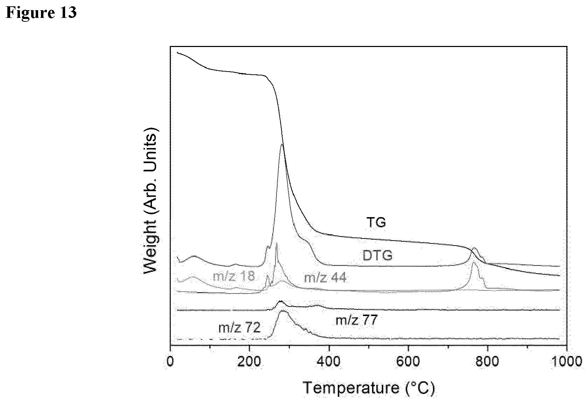

The thermal stability of pristine compound **NCP-[Cu₂(IBA)₂(H₂O)₄]ₙ·6*ₙ*H₂O** and **NCP-[Cu₂(IBA)₂(H₂O)₄]ₙ·**previously heated at 100 °C has been done to check **(****Figure 13****)** the number of lost water molecules that can cause its thermochromism. In the pristine compound, all water molecules were lost at 254 °C in three stages (obs. 14.97%; calc. 15.2%) starting at 100 °C with the loss of the first one. Once compound **NCP-[Cu₂(IBA)₂(H₂O)₄]ₙ·6*ₙ*H₂O** changed its color to green was again thermally analyzed to confirm that a lower weight-loss of water than the above thermogram was produced. In this thermogram, the initial stage had a loss of 6.5 % versus 15 %. This percentage could indicate the loss of three solvation water molecules, and the rest stages seem to be like the before one.

See **Figure 13** showing the thermal stability of compound **NCP-[Cu₂(IBA)₂(H₂O)₄]ₙ** after being previously heated at 100 °C. Black and red lines represent the weight (TG) and derivate weight (DTG), respectively, showing the stages of the losses produced. Multicolor-labeled lines represent the ion current associated with each mass lost in each stage.

### Example 1.5. Copper release and catalytic activity

### Example 1.5.1. Sample preparation for copper release assays

0.05 g of compound **NCP-[Cu₂(IBA)₂(H₂O)₄]ₙ·6*ₙ*H₂O** was dispersed in 5 ml of deionized water. The sample's dispersion was left shaking at 140 rpm and room temperature. After 24 h, the sample was centrifuged in a High-Speed Brushless Centrifuge MPW-350R for 2 h at 2500 rpm. The supernatant was filtered and analyzed. Then the medium was replenished with another 5 ml of fresh water, and the sample was again shaken. This procedure was repeated until seven days (t=1, 3, 6, and 7 days). Supernatant from single-water samples was analyzed by TXRF.

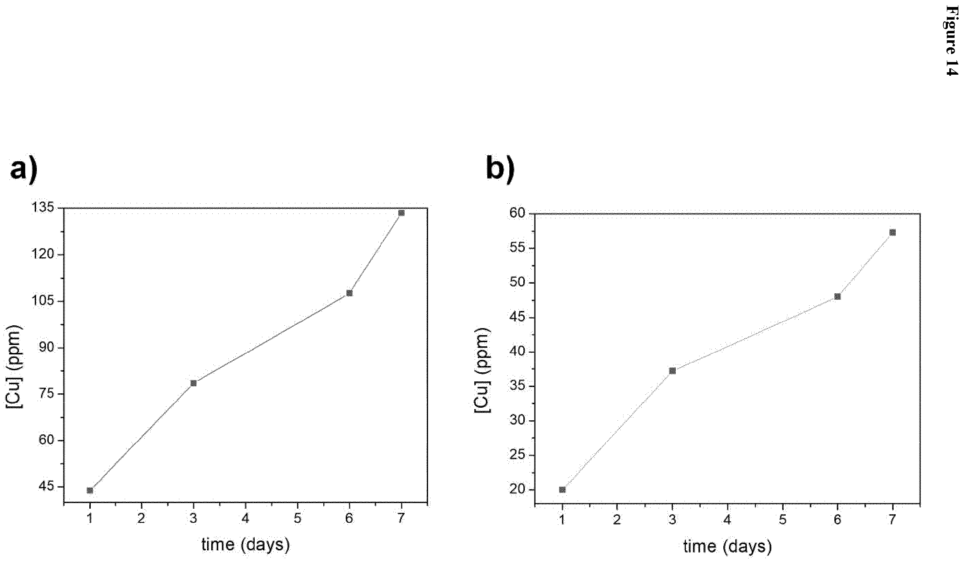

See **Figure 14** showing the copper concentration of compound **NCP-[Cu₂(IBA)₂(H₂O)₄]ₙ·6*ₙ*H₂O** in water (a) and PBS (b) after carrying out copper release assays.

### Example 1.5.2. Peroxidase-like activity measurement

The catalytic activity of the **NCP-[Cu₂(IBA)₂(H₂O)₄]ₙ·6*ₙ*H₂O** compound (3 mg/ml) in the presence of H₂O₂ (0.05 ml) as a peroxidase enzyme was investigated through the oxidation of TMB (0.05 ml, 10 mM). The experiments were carried out in phosphate-buffered saline (PBS) (pH 7.5, 50 mM) and monitored spectrophotometrically (JASCO V-550 UV/vis spectrometer) from 240 nm to 700 nm and the activity at 440 nm for 10 minutes.

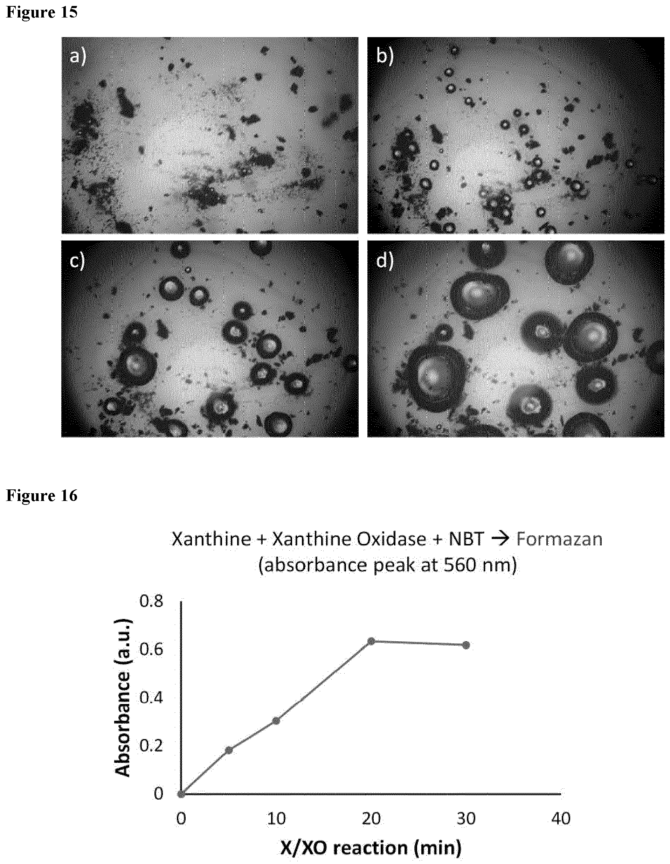

See **Figure 15** showing oxygen bubbles generation when **NCP-[Cu₂(IBA)₂(H₂O)₄]ₙ·6*ₙ*H₂O** is exposed to H₂O₂ 30 % for a) t=0, b) t=10 s, c) t=20 s and d) t=30 s.

### Example 1.4.4. SOD-like activity measurement

The SOD activity of the **NCP-[Cu₂(IBA)₂(H₂O)₄]ₙ·6*ₙ*H₂O** was evaluated employing the nitroblue tetrazolium (NBT) method, using xanthine/xanthine oxidase as a source of superoxide anion. The superoxide anion promotes the reduction of NBT to formazan, which can be evaluated spectrophotometrically at 560 nm.

All stock solutions were made fresh on the day of use. Xanthine solution (50mM) was prepared in PB buffer (50mM, pH 8) containing 0.3 M of sodium hydroxide. NBT (2.3 mM) was prepared in PB buffer (50mM, pH 8). The reaction medium had PB buffer (50mM, pH 8), 3mM xanthine, 0.02 U/mL xanthine oxidase, 25.5 µM NBT and the test sample at different concentrations in a total volume of 100 µL. A control experiment was conducted by mixing all reagents, except that the test sample was substituted with an equal volume of PB buffer.

The change in absorption was monitored over 30 minutes. The absorption reached a maximum after 20 minutes of reaction and was used to calculate the IC₅₀. The experiments were done in triplicates, and the IC₅₀ was calculated from a linear regression analysis.

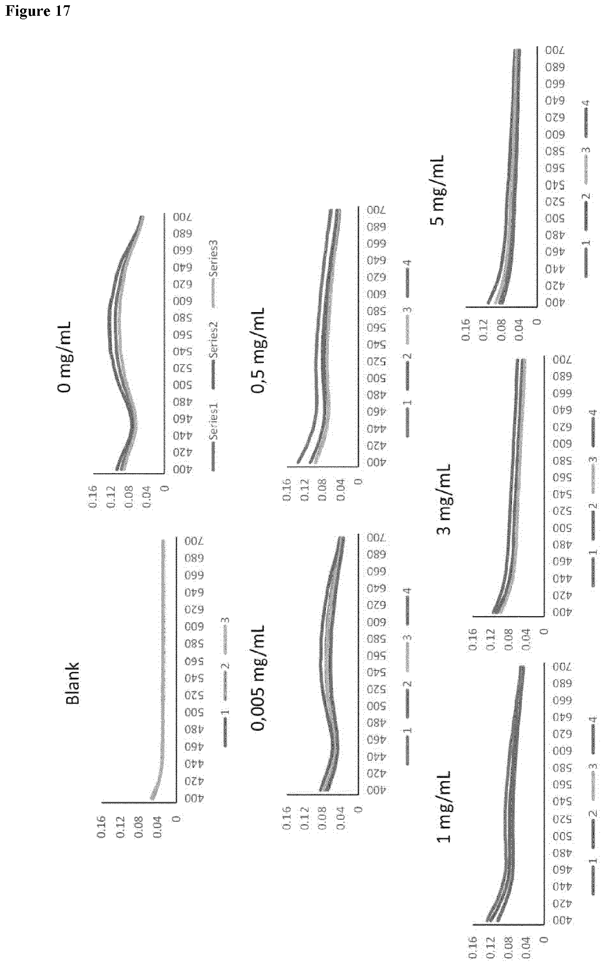

See **Figure 16** showing the absorbance measurement against reaction time when the absorption reaches a maximum and **Figure 17** showing measurements of NBT absorption spectra at 560 nm upon 20 min of reaction to different concentrations of **NCP-[Cu₂(IBA)₂(H₂O)₄]ₙ·6*ₙ*H₂O.**

### Example 1.6. Antibacterial Experiments

The antibacterial activity of the compound was tested by agar diffusion against different microorganisms (*E. coli DH5*α*, E. coli Bl21, E. coli K12, E. coli XL1Blue, P. fluorescens, S. spiritovirum, B. cereus, B. circulans, B. subtilis, S. epidermis, A. faecalis, D. radiodurans* and *L. lactis*). Briefly, a standardized inoculum of the microorganism is swabbed onto the surface of LB-agar plate. Normally, filter paper disks are impregnated with a standardized concentration of an antimicrobial agent placed on the surface. The size of the zone of inhibition around the disk is measured after overnight incubation at 37 °C. In our case, after swabbed of the microorganism, we have directly added the antimicrobial agent or compounds onto the plate, so the diameter of the inhibition zone properly describes the antimicrobial potency of the compounds. We have used ampicillin (2 µl at 3 mg/ml), kanamycin (2 µl at 3 mg/ml), **NCP-[Cu₂(IBA)₂(H₂O)₄]ₙ·6*ₙ*H₂O** (1-20 µl at 3 mg/ml), **NCP-[Cu₂(IBA)₂(H₂O)₄]ₙ·6*ₙ*H₂O**:H₂O₂ (1-20 µl: 1 µl) as antimicrobial agents and CuSO₄ (20 µl at 3 mg/ml) or H₂O₂ as control.

The antimicrobial activities of **NCP-[Cu₂(IBA)₂(H₂O)₄]ₙ·6*ₙ*H₂O,** and **NCP-[Cu₂(IBA)₂(H₂O)₄]ₙ·6*ₙ*H₂O@gelatin** in presence or absence of H₂O₂, the minimal inhibitory concentration (MIC), and zone of inhibition against *Sphingobacterium or E. coli* were studied by using optical density at 600nm and the colony count method ²

To determinate the growth curve of bacteria, the compound **NCP-[Cu₂(IBA)₂(H₂O)₄]ₙ·6*ₙ*H₂O** or **NCP-[Cu₂(IBA)₂(H₂O)₄]ₙ·6*ₙ*H₂O@gelatin** was dispersed at different concentrations (0.02-0.16 mg/ml) into sterilized tubes with liquid broth medium (LB) and 50µL of H₂O₂ 25mM in water. Briefly, 50µL of well-cultivated bacterial solution of *Sphingobacterium* (10⁶ CFU/mL, overnight) was added to each tube with 5 mL of LB and different concentrations of **NCP-[Cu₂(IBA)₂(H₂O)₄]ₙ·6*ₙ*H₂O** compound plus H₂O₂ (25 mM) and were left 24 h to shake at 37 °C. Absorbance measurements of the samples were recorded at 600 nm each hour. In the case of MIC assay, when the OD₆₀₀ₙₘ reached 0.5 value, was added the NCP-**[Cu₂(IBA)₂(H₂O)₄]ₙ·6*ₙ*H₂O** at different concentrations, and H₂O₂ (25 mM).

The minimal inhibitory concentration (MIC) of the compound NCP-**[Cu₂(IBA)₂(H₂O)₄]ₙ·6*ₙ*H₂O** without and with H₂O₂ was assayed. Briefly, 25µL of well-cultivated bacterial solution of *Sphingobacterium* (10⁶ CFU/mL) was added to 5 mL of M-H with shaking at 37 °C until it reached a OD₆₀₀ₙₘ= 0.5. Then, **NCP-[Cu₂(IBA)₂(H₂O)₄]ₙ·6*ₙ*H₂O** alone at different concentrations (control) or with 50 µL of H₂O₂ at 25 mM were added. The absorbance was tested with the time at 600 nm. The 25 µL of supernatant of two samples (*Sphingobacterium* broth and the same broth with 0.16 mg/ml of NCP-**[Cu₂(IBA)₂(H₂O)₄]ₙ·6*ₙ*H₂O** plus 50 µL H₂O₂ (25 mM)) was extracted from each tube after 24 h and was uniformly spread over agar nutrient plates.

**Table 1.** Strains tested against compound **NCP-[Cu₂(IBA)₂(H₂O)₄]ₙ·6*ₙ*H₂O** and its minimum inhibitory concentration (MIC).

**Table 1**

| | **MIC (µg/ml)** | | | |
|---|---|---|---|---|
| ***Strains*/**Gram (+/-) | H₂IBA ligand | NCP-**[Cu₂(IBA)₂(H₂O)₄]ₙ·6*ₙ*H₂O** | **NCP-[Cu₂(IBA)₂(H₂O)₄]ₙ·6*ₙ*H₂O +** H₂O₂ | Amp |
| *Escherichia coli DH5a* (-) | >3 | >3 | >3 | <0.3 |
| *Escherichia coli Bl21*(*-*) | >3 | >3 | >3 | <0.3 |
| *Escherichia coli K12*(*-*) | >3 | >3 | >3 | <0.3 |
| *Escherichia coli XL1Blue*(*-*) | >3 | >3 | >3 | <0.3 |
| *Pseudomonas aeruginosa* (-) | >3 | >3 | >3 | <0.3 |
| *Pseudomonas fluorescens*(*-*) | >3 | >3 | >3 | <0.3 |
| *Sphingobacterium spiritovirum*(-) | >3 | >3 | <0.1 | >1 |
| *Bacillus cereus(+)* | >3 | <3 | <1 | <0.3 |
| *Bacillus circulans* (+) | >3 | >3 | >3 | <0.3 |
| *Bacillus subtilis* (+) | >3 | >3 | >3 | <0.3 |
| *Staphylococcus epidermis* | >3 | >3 | >3 | <0.3 |
| *Alcaligenes faecalis* (-) | >3 | >3 | <1 | >1 |
| *Deinococcus radiodurans* (+) | >3 | >3 | >3 | <0.3 |
| *Lactococcus lactis* (+) | >3 | >3 | >3 | <0.3 |

### Example 2. Results

### Example 2.1. Chemical and morphological characteristics of NCP-[Cu₂(IBA)₂(H₂O)₄]ₙ·6ₙH₂O

An essential advantage in the synthesis of this CP is the possibility of obtaining it in different phases and sizes by slightly modifying the synthetic conditions. That is, the direct reaction between H₂IBA deprotonated with NaOH can generate compound **[Cu₂(IBA)₂(H₂O)₄]ₙ·6*ₙ*H₂O** as single microcrystals **(scheme 1a),** as polycrystalline, as a colloid formed by nanofibers **NCP-[Cu₂(IBA)₂(H₂O)₄]ₙ·6*ₙ*H₂O (Scheme 1b)** or as metal organic gel **(NCP-[Cu₂(IBA)₂(H₂O)₄]ₙ·6*ₙ*H₂O@MOG) (Scheme 1c)** depending on the starting copper (II) salt and the application of different times of ultrasounds.

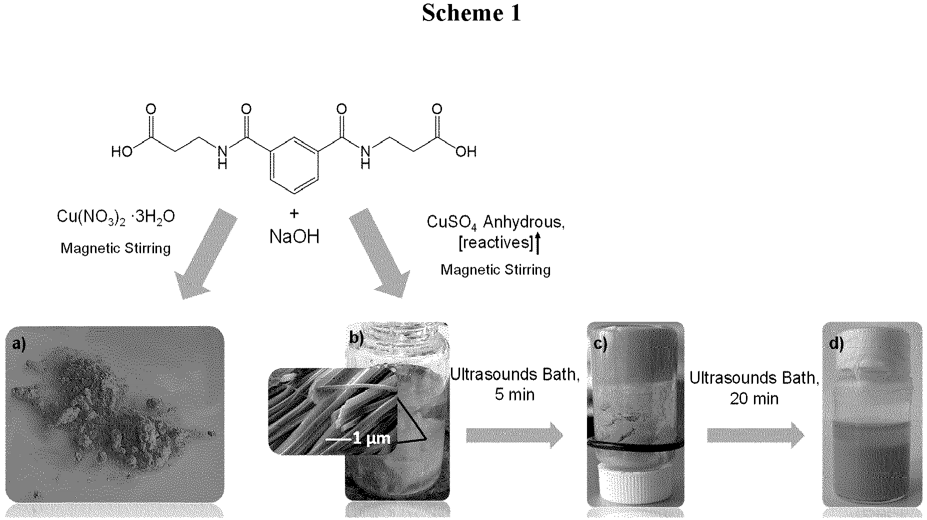

### Different conditions to obtain compound [Cu₂(IBA)₂(H₂O)₄]ₙ·6ₙH₂O as bulk material (a), NCP-[Cu₂(IBA)₂(H₂O)₄]ₙ·6ₙH₂O fibers (b), Metal-organic gel, NCP-[Cu₂(IBA)₂(H₂O)₄]ₙ·6ₙH₂O@MOG (c), or nanocrystals, NCP-[Cu₂(IBA)₂(H₂O)₄]ₙ·6ₙH₂O (d).

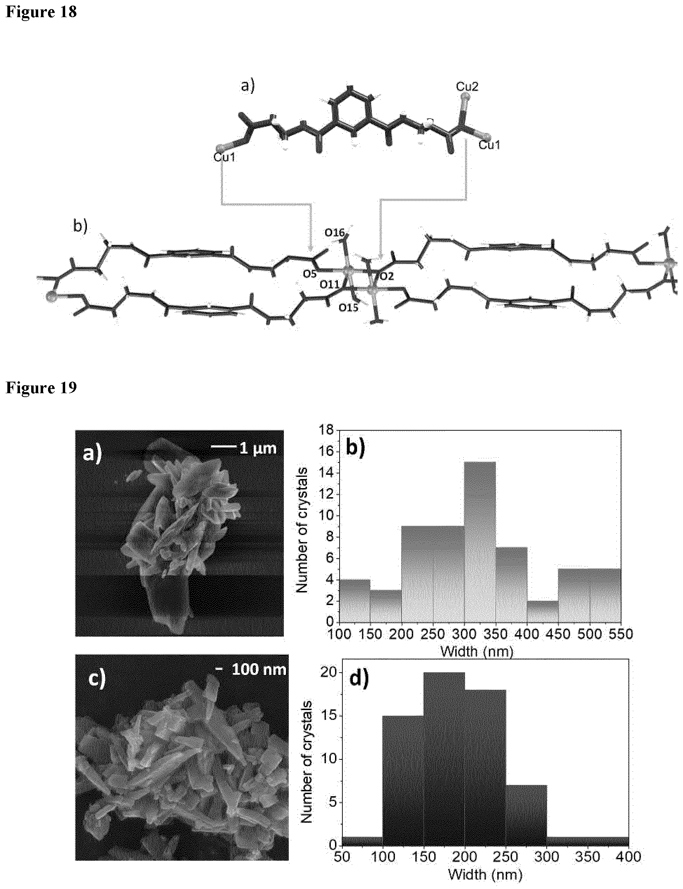

Structurally speaking, compound **[Cu₂(IBA)₂(H₂O)₄]ₙ·6*ₙ*H₂O** is a 2D coordination polymer that crystallizes in the monoclinic Pc space group. It consists of a metallic core form of dimeric species in which each metal presents an irregular square pyramidal geometry **(****Figure 18****)** involving two oxygen atoms from one syn-syn bridging (Cu···O2 1.94 Å) and one from monodentate coordinated (Cu···O5 1.92 Å) ligands (IBA2-) and two oxygen atoms from two water molecules (Cu···O15 1.96 and Cu···O16 1.98 Å) in the basal plane. The apical position, with a significantly longer bond distance (Cu···O11 2.36 Å) is occupied by one oxygen atom from the other syn-syn bridging coordinated ligand.

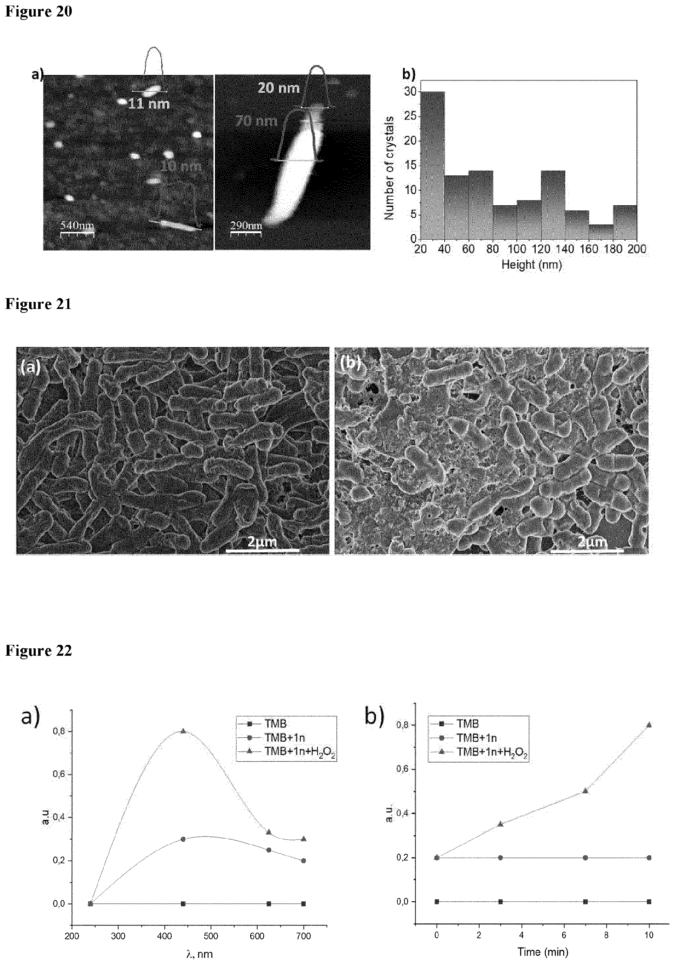

As ultrasounds are used to assist sol-gel transitions, a bottom-up approach using CuSO₄ instead of Cu(NO₃)₂ and sonication allows the formation of unstable gel **(NCP-[Cu₂(IBA)₂(H₂O)₄]n·6*ₙ*H₂O@MOG) (Scheme 1c),** which is transformed into a colloid formed by the corresponding nanocrystals **(NCP-[Cu₂(IBA)₂(H₂O)₄]n·6*ₙ*H₂O) (Scheme 1d)** by the use of longer sonication times. The morphology and dimensions of both phases were studied by SEM and AFM **(****Figures 19** and **20****).** The average width for **[Cu₂(IBA)₂(H₂O)₄]ₙ·6*ₙ*H₂O** and **NCP-[Cu₂(IBA)₂(H₂O)₄]ₙ·6*ₙ*H₂O** are 354±170 nm and 170±60 nm, respectively **(****Figure 19****),** while the average height for **NCP-[Cu₂(IBA)₂(H₂O)₄]ₙ·6*ₙ*H₂O** is 46±20 nm **(****Figure 20****).**

Compound **NCP-[Cu₂(IBA)₂(H₂O)₄]ₙ·6*ₙ*H₂O** is stable at physiological pHs (between pHs 3 and 8) for long periods (1 year). Additionally, it shows thermal stability up to 254 °C. It loses all the solvation water molecules at this temperature, and the coordinated ligands decompose mainly into CO₂(g). It should be mentioned that at temperatures above 100 °C, the blue color compound **(NCP-[Cu₂(IBA)₂(H₂O)₄]ₙ·6*ₙ*H₂O,** reversibly transforms into a new less crystalline green color phase, as a consequence of the loss of the first solvated water molecules. The magnetic behavior of this compound, which shows antiferromagnetic coupling, has also been studied.

### Example 2.2. Antibacterial test of NCP-[Cu₂(IBA)₂(H₂O)₄]ₙ·6ₙH₂O

To determine the copper concentrations produced when compound **NCP-[Cu₂(IBA)₂(H₂O)₄]ₙ·6*ₙ*H₂O** is in suspension, release assays of the compound in deionized water were carried out. Considering the slow Cu(II) release of the system (45 ppm at 24 h), its nanometric dimensions, synthesis in water a room temperature in just one step, and the recent results that have demonstrated the possibility of related coordination polymers to act as artificial enzymes, its antimicrobial activity was explored. Different bacteria strains **(Table 1)** such as Gram-positive (*Bacillus, Deinococcus, Lactococcus Lactis*) or Gram-negative (*Escherichia, Pseudomonas, Sphingobacterium, Alcaligenes*) have been exposed against compound **NCP-[Cu₂(IBA)₂(H₂O)₄]ₙ·6*ₙ*H₂O.** Compound **NCP-[Cu₂(IBA)₂(H₂O)₄]ₙ·6*ₙ*H₂O** itself showed growth inhibition in *Sphingobacterium* and *A. faecalis,* although minimum inhibitory concentrations (MIC) three times higher than ampicillin (Amp) used as control are required **(Table 1).**

The peroxidase-like activity of this compound is revealed in the presence of H₂O₂ at low concentrations (25 mM), generating reactive oxygen species and inhibiting the growth of *A. faecalis, B. cereus* (to a lesser extend), and *Sphingobacterium* remarkably (<0.1 mg/ml **NCP-[Cu₂(IBA)₂(H₂O)₄]ₙ·6*ₙ*H₂O** is needed *versus* >1 mg/ml Ampicillin) **(Table 1).** *Sphingobacterium* bacterial growth was also measured by the optical density method at 600 nm (OD₆₀₀). The minimal inhibition concentrations (MICs) of **NCP-[Cu₂(IBA)₂(H₂O)₄]ₙ·6*ₙ*H₂O** were at 300 µg/mL and at the same concentration of **NCP-[Cu₂(IBA)₂(H₂O)₄]ₙ·6*ₙ*H₂O** plus H₂O₂ nothing of growing of the bacteria after overnight at 37 °C was detected **(Table 2).**

**Table 2**

| **Compound/Bacteria** | **OD₆₀₀ₙₘ, 24h 37°C** |
|---|---|
| NCP-[Cu₂(IBA)₂(H₂O)₄]ₙ·6*ₙ*H₂O | |
| ***Sphingobacterium*** | 2,67 |
| ***Sphingobacterium*** + ***5 µL Kanamycine* (3mg/ml)** | 2,44 |
| ***Sphingobacterium* + 1 µl H₂O₂ (25mM)** | 2,34 |
| ***Sphingobacterium* + 100 µL CuSO₄ (3mg/ml)** | 1,35 |
| ***Sphingobacterium* + 100 µL** NCP-[Cu₂(IBA)₂(H₂O)₄]ₙ·6*ₙ*H₂O + **50 µl H₂O₂** | 0,05 |

### OD values at 600 nm of the Sphingobacterium

*in the presence of kanamycin antibiotic, H₂O₂,* **NCP-[Cu₂(IBA)₂(H₂O)₄]ₙ·6*ₙ*H₂O** *and* **NCP-[Cu₂(IBA)₂(H₂O)₄]ₙ·6*ₙ*H₂O** *plus H₂O₂.*

Furthermore, the changes of *Sphingobacterium* morphology affected by **NCP-[Cu₂(IBA)₂(H₂O)₄]ₙ·6*ₙ*H₂O**+H₂O₂ were obtained using FESEM **(****Figure 22****).** Compared with control, the bacterial surface was severely damaged by the presence of **NCP-[Cu₂(IBA)₂(H₂O)₄]ₙ·6*ₙ*H₂O** + H₂O₂ upon 24 h, probably by the generation of HO· that could oxidize the cell membranes.

### Example 2.3. Peroxidase mimicking activity of NCP-[Cu₂(IBA)₂(H₂O)₄]ₙ·6ₙH₂O

The excellent intrinsic peroxidase-like activity of **NCP-[Cu₂(IBA)₂(H₂O)₄]ₙ·6*ₙ*H₂O** was evaluated by oxidizing chromogenic peroxidase substrate 3,3',5,5'-tetramethylbenzidine (TMB) to form a blue-colored product under the assistance of H₂O₂ **(****Figure 22****).** TMB itself is colourless and displays no absorbance **(****Figure 22a** and **Figure 22b****).** As shown in **Figure 22a****,** a strong absorption was observed in the group of **TMB** + **NCP-[Cu₂(IBA)₂(H₂O)₄]ₙ·6*ₙ*H₂O** + **H₂O₂,** indicating that **NCP-[Cu₂(IBA)₂(H₂O)₄]ₙ·6*ₙ*H₂O** possessed excellent catalytic activity. Moreover, a time-dependent change in the TMB absorbance was recorded by a UV-vis spectrophotometer at 440 nm **(****Figure 22a****).**

### Example 2.4. Superoxide dismutase (SOD) mimicking activity of NCP-[Cu₂(IBA)₂(H₂O)₄]ₙ·6ₙH₂O

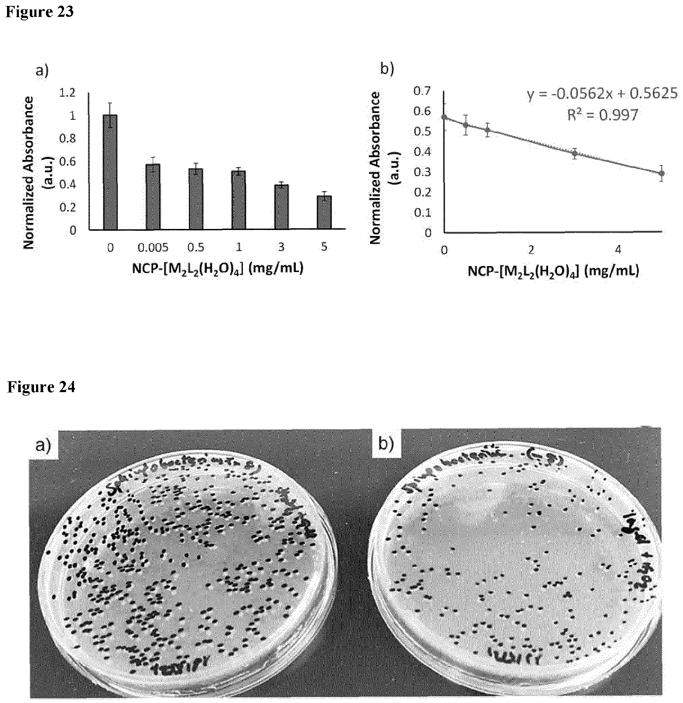

In order to study if this **NCP-[Cu₂(IBA)₂(H₂O)₄]ₙ·6*ₙ*H₂O** can be a candidate as an antioxidant mimic enzyme, useful in the treatment of disorders related to oxidative stress, it was decided to study its activity as a superoxide dismutase (SOD). To evaluate this SOD-like activity, an assay with the nitroblue tetrazolium (NBT) was performed to quantify the **NCP-[Cu₂(IBA)₂(H₂O)₄]ₙ·6*ₙ*H₂O** enzymatic activity. The kinetics of the reaction between NBT and the xanthine/xanthine oxidase (x/xo) system was measured to establish the time at which the absorbance reaches its maximum value at a wavelength of 560 nm. Triplicate NBT spectra were then performed with different concentrations of **NCP-[Cu₂(IBA)₂(H₂O)₄]ₙ·6*ₙ*H₂O** in a stepwise manner (0, 0.005, 0.5, 1, 1, 3, and 5 mg/ml), observing a continuous decrease of the signal as the concentration of **NCP-[Cu₂(IBA)₂(H₂O)₄]ₙ·6*ₙ*H₂O** increases and disappearing completely at a concentration of 3 mg/ml. Nearly 80 % of the superoxides were removed from a concentration of 5 mg/ml of **NCP-[Cu₂(IBA)₂(H₂O)₄]ₙ·6*ₙ*H₂O** and IC₅₀ was calculated **(****Figure 23****)** to establish the concentration of **NCP-[Cu₂(IBA)₂(H₂O)₄]ₙ·6*ₙ*H₂O** that inhibits the rate of NBT reduction by 50 % (1.1 mg/ml).

### Example 2.5. Catalase mimicking activity of NCP-[Cu₂(IBA)₂(H₂O)₄]ₙ·6ₙH₂O

Catalase is responsible for the catalytic decomposition of hydrogen peroxide by means of its disproportionation reaction into nontoxic dioxygen and water. The catalytic activity of **NCP-[Cu₂(IBA)₂(H₂O)₄]ₙ·6*ₙ*H₂O** towards the decomposition of hydrogen peroxide has been investigated in water at 25 °C. In the beginning, **NCP-[Cu₂(IBA)₂(H₂O)₄]ₙ·6*ₙ*H₂O suspension** was dark blue coloured, but after the addition of 30% (v/v) hydrogen peroxide, it became faded yellow coloured rapid evolution of gas was observed. It can be easily understood that the evolved gas is oxygen, and it comes from the catalytic decomposition of hydrogen peroxide solution **(****Figure 15****).**

### Example 2.6. Chemical and morphological characteristics of NCP-[Cu₂(IBA)₂(H₂O)₄]ₙ·6ₙH₂O@gelatin and its antibacterial activity

Like the metal organic gel **(NCP-[Cu₂(IBA)₂(H₂O)₄]ₙ·6*ₙ*H₂O@MOG)** obtained, it is not stable at physiological pH (only around pH 3), and in order to process this material in a moldable and comfortable way, gelatin as a biocompatible organic matrix has been used to create a gel-like composite allowing the release and diffusion of **NCP-[Cu₂(IBA)₂(H₂O)₄]ₙ·6*ₙ*H₂O** during the incubation stage thanks to its great solubility in water at 37 °C. With compound **NCP-[Cu₂(IBA)₂(H₂O)₄]ₙ·6*ₙ*H₂O** in this format, improved manufacture and handling of the material is achieved, facilitating its application in *in vitro* tests regarding bulk material.

The antibacterial efficacy of the **NCP-[Cu₂(IBA)₂(H₂O)₄]ₙ·6*ₙ*H₂O@gelatin** was checked against *Sphingobacterium* by the colony count method. The number of grown colonies of *Sphingobacterium* overnight at 37°C was 490 colonies while in the presence of **NCP-[Cu₂(IBA)₂(H₂O)₄]ₙ·6*ₙ*H₂O@gelatin** plus H₂O₂ (25mM) it was half **(****Figure 24****).**

## Claims

1. A composition comprising:
a) A coordination polymer which in turn comprises a polymeric crystalline structure with a repeating core **characterized by** *Formula I:* **M**₂**L**₂(H₂O)₄ wherein:
i. **M** is copper (II) metal ion,
ii. **L** is an aromatic pseudo-peptide **characterized by** *Formula II:* H₂**I**-bis-**amino acid** or *Formula III:* H₂**T**-bis-**amino acid,** wherein **T** is Terephthaloyl, **I** is Isophthaloyl, and the **amino acid** is a L or D amino acid; and
b) H₂O₂.

2. Composition, according to claim 1, wherein L is an aromatic pseudo-peptide selected from the group comprising: H₂**T**-bis-L-aspartate, H₂**T**-bis-D-aspartate; H₂**T**-bis-L-alanine, H₂**T**-bis-D-alanine; H₂**T**-bis-L-valine, H₂**T**-bis-D-valine, H₂**T**-bis-L-isoleucine, H₂**T**-bis-D-isoleucine, H₂**T**-bis-L-leucine, H₂**T**-bis-D-leucine, H₂**T**-bis-L-phenylalanine, H₂**T**-bis-D-phenylalanine, H₂**T**-bis-β-alanine, H₂**T**-bis- β-alanine; H₂**I**bis β-alanine H₂**I**-bis-L-aspartate, H₂**I**-bis-D-aspartate; H₂**I**-bis-L-alanine, H₂**I**-bis-D-alanine; H₂**I**-bis-L-valine, H₂**I**-bis-D-valine, H₂**I**-bis-L-isoleucine, H₂**I**-bis-D-isoleucine, H₂**I**-bis-L-leucine, H₂**I**-bis-D-leucine, H₂**I**-bis-L- phenylalanine, H₂**I**-bis-D-phenylalanine, H₂**I**-bis-β-alanine or H₂**T**-bis- β-alanine, wherein **T** is Terephthaloyl, **I** is Isophthaloyl.

3. Composition, according to any of the claims 1 or 2, comprising:
a) A coordination polymer which in turn comprises a polymeric crystalline structure with a repeating core **characterized by** *Formula I:* **M**₂**L**₂(H₂O)₄ wherein:
i. **M** is copper (II) metal ion,
ii. **L** is H₂-Isophthaloyl-bis-β-alanine; and
b) H₂O₂.

4. Composition, according to any of the claims 1 to 3, **characterized in that** the polymeric crystalline structure of *Formula I* is in the form of a composite material wherein the polymeric crystalline structure of *Formula I* is embedded in an organic matrix.

5. Composition, according to any of the claims 1 to 4, wherein the organic matrix is selected from: polyethylene glycol, agarose, polyacrylamide, cellulose, fiber, gelatin or chitosan.

6. Composition, according to any of the claims 1 to 5, **characterized in that** the polymeric crystalline structure of *Formula I* is in the form of a mouldable gelatin.

7. Pharmaceutical composition comprising the composition of any of the claims 1 to 6 and, optionally, pharmaceutically acceptable excipient or carriers.

8. Use of the composition of any of the claims 1 to 6 for assessing the presence of a catalase-positive microorganism in a test sample, by means of the identification of the inhibition of the microorganism, wherein the composition exerts a growth-inhibiting effect on the microorganism.

9. Use of the composition of any of the claims 1 to 6, according to claim 8, for assessing the presence of a catalase and oxidase-positive aerobic or anaerobic microorganism in a test sample, by means of the identification of the inhibition of the microorganism, wherein the composition exerts a growth-inhibiting effect on the microorganism.

10. Use of the composition of any of the claims 1 to 6, according to any of the claims 8 or 9, wherein the catalase-positive microorganism is a bacterium pertaining to a genus selected from: *Sphingobacterium, Alcaligenes* or *Bacillus.*

11. Use of the composition of any of the claims 1 to 6, according to any of the claims 8 to 10, wherein the catalase-positive microorganism is a bacterium selected from: *Sphingobacterium spiritivorum, Alcaligenes faecalis* or *Bacillus cereus.*

12. Method for assessing the presence of a catalase-positive microorganism in a test sample by means of the identification of the inhibition of the microorganism, which comprises:
a) Spreading the composition of any of the claims 1 to 6 over the test sample, and
b) Wherein the identification of a zone of inhibition in the test sample is indicative of the inhibition of the microorganism.

13. Method, according to claim 12, for assessing the presence of a catalase and oxidase aerobic or anaerobic microorganism in a test sample by means of the identification of the inhibition of the microorganism, which comprises:
a) Spreading the composition of any of the claims 1 to 6 over the test sample, and
b) Wherein the identification of a zone of inhibition in the test sample is indicative of the inhibition of the microorganism.

14. Method, according to any of the claims 12 or 13, wherein the catalase-positive microorganism is a bacterium pertaining to a genus selected from: *Sphingobacterium, Alcaligenes* or *Bacillus,* preferably a bacterium selected from: *Sphingobacterium spiritivorum, Alcaligenes faecalis* or *Bacillus cereus.*

15. Pharmaceutical composition of claim 7 for use as a medicament, preferably in the treatment of a bacterial infection.
